# EUROPEAN PATENT SPECIFICATION

(11) **EP 4 694 771 B1**
(45) Date of publication and mention of the grant of the patent: **14.10.2026**
(21) Application number: 24719487.1
(22) Date of filing: 05.04.2024
(51) Int. Cl.: A61B 6/00, A61B 6/58

(54) **UWB-BASED MOBILE X-RAY POSITIONING**
UWB-BASIERTE MOBILE RÖNTGENPOSITIONIERUNG
POSITIONNEMENT DE RAYONS X MOBILE BASÉ SUR UWB

(30) Priority: 10.04.2023 RU 2023108952
(43) Date of publication of application: 18.02.2026
(73) Proprietor: Koninklijke Philips N.V., 5656 AE Eindhoven (NL)
(72) Inventor: EFITOROV, Aleksandr, 5656 AG Eindhoven (NL); SCHULZ, Heinrich, 5656 AG Eindhoven (NL); RENISCH, Steffen, 5656 AG Eindhoven (NL)
(74) Representative: Philips Intellectual Property & Standards
(86) International application number: PCT/EP2024/059366
(87) International publication number: WO 2024/213482

(56) References cited:
- US-A1- 2009 257 564
- US-A1- 2014 112 439
- US-A1- 2015 049 863
- US-A1- 2016 183 909

## Description

### FIELD OF THE INVENTION

The present invention relates to a system for positioning an X-ray source and an X-ray detector of a mobile X-ray imaging device, a mobile X-ray imaging comprising a corresponding system, and a method for positioning an X-ray source and an X-ray detector of a mobile X-ray imaging device.

### BACKGROUND OF THE INVENTION

X-Ray machines are one of the most common patient monitoring devices, providing fast imaging of the patient, which is an advantage, especially in the cases of a critical patient condition. The Covid-19 pandemic has highlighted the need for mobile X-ray machines to estimate lung damage and track disease progression. The through-glass imaging implementation of mobile X-ray machines in the hospitals has reduced the size of the pandemic/ red zones in the hospitals as it eliminated the need for transportation of infected patients from their isolated places through the hospital to a stationary X-ray imaging device. Since there is no need to organize a visit of an infected patient at a radiologist, hospital costs and infection risk can be reduced. However, obtaining a high-quality image from a mobile X-ray detector for patients in intensive care unit is a solely challenging task, and additional obstacles like glass windows and increased distance between X-ray source and detector makes it more difficult to acquire a high quality image of the patient. Due to the free mutual arrangement of the X-ray source and the X-ray detector, a precise control of their location for high quality image acquisition is required.

US 2016/183909 A1 describes an X-ray imager having a navigation-aid subsystem including one or more transmitters, one or more receivers and one or more reflectors. A radio signal is trnasmitted by a transmitter, is then reflected off a reflector and is then received at a receiver. The received signal is resolved into positional correction information that can be used to guide a motion of the imager's tube or detector.

The inventors of the present invention have thus found that it would be advantageous to have a system and a method for positioning an X-ray source and an X-ray detector of a mobile X-ray imaging device that at least partially solves these problems and provides a reliable and precise positioning of the source in relation to the detector that even can be implemented when source and detector are separated from each other, for example, by a glass window.

### SUMMARY OF THE INVENTION

It is an object of the present invention to provide an improved positioning of an X-ray source and an X-ray detector of a mobile X-ray imaging device.

The object of the present invention is solved by the subject matter of the independent claims, wherein further embodiments are incorporated in the dependent claims.

The described embodiments similarly pertain to the system for positioning an X-ray source and an X-ray detector of a mobile X-ray imaging device, the mobile X-ray imaging comprising a corresponding system, and the method for positioning an X-ray source and an X-ray detector of a mobile X-ray imaging device. The embodiments described further may be combined in any possible way. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method embodiment, unless explicitly mentioned to the contrary hereinafter.

According to a first aspect of the invention, there is provided a system for positioning an X-ray source and an X-ray detector of a mobile X-ray imaging device. The system comprises a source module configured to be mechanically connected to the X-ray source of the mobile X-ray imaging device, the source module comprising a plurality of ultra-wideband transceivers, and a detector module configured to be mechanically connected to the X-ray detector of the mobile X-ray imaging device, the detector module comprising a plurality of ultra-wideband transceivers. The system comprises further a control unit configured for communicating with the ultra-wideband transceivers and for determining mutual distances of each of the plurality of ultra-wideband transceivers of the source module to each of the plurality of ultra-wideband transceivers of the detector module, and an interface unit configured for displaying data corresponding to the determined mutual distances.

Thus, the proposed invention implements smart guided mobile X-ray positioning, and helps a technologist to locate the mobile X-ray collimator head in relation to the detector plate to obtain appropriate high-quality X-ray images of a patient. Ultra-wideband transceivers can be mounted in preferably removable plastic frames, with a first frame being connected to an X-ray collimator head, and a second frame being connected to the X-ray detector. Preferably, at least three ultra-wideband transceivers are connected to the X-ray source, and at least three ultra-wideband transceivers are connected to the X-ray detector.. Preferably, the transceivers can be located at detector corners to determine the position of the detector plane. A computing unit controlling the ultra-wideband transceivers determines the relative positions of the ultra-wideband transceivers with respect to each other, and in particular with respect to the ultra-wideband transceivers located at the respective other one of source and detector. If the mutual distances of one transceiver to the other transceivers are known, a geometrical triangulation can be solved and the exact relative position and orientation of the X-ray source with respect to the X-ray detector can be calculated. By comparison with a predefined reference position ensuring optimal imaging and focused irradiation of the X-ray detector, corrections for the current position of the X-ray source and collimator can be proposed. An interface unit can be used to inform the technician and provide instructions how to adjust the position of the X-ray source. For example, the proposed system can suggest the technician to move a head of the X-ray source horizontally and/or vertically, and/or to adjust an orientation of the X-ray head to observe the desired area of the body. In addition, the technician can be instructed to shift and/or tilt the X-ray detector of the mobile X-ray imaging device.
Thus, the present invention allows obtaining precisely adjusted X-ray images received from mobile detectors. It may eliminate the need for snapshot re-acquiring and increases the accuracy of a diagnosis.

In an embodiment of the invention, each of the ultra-wideband transceivers is configured for determining a mutual distance to at least one another ultra-wideband transceiver.

In an embodiment of the invention, the plurality of ultra-wideband transceivers of the source module comprises at least three ultra-wideband transceivers, and the plurality of ultra-wideband transceivers of the detector module comprises at least three ultra-wideband transceivers. In a preferred embodiment, the X-ray detector is a rectangular detector, and one ultra-wideband transceiver is positioned at every corner of the detector. Thus, four UWB transceivers are positioned at the detector.

In an embodiment of the invention, the source module comprises a source frame configured for receiving the plurality of ultra-wideband transceiver, and the source frame is configured to be attached to the X-ray source of the mobile X-ray imaging device.

In an embodiment of the invention, the detector module comprises a detector frame configured for receiving the plurality of ultra-wideband receivers, and the detector frame is configured for receiving the X-ray detector of the mobile X-ray imaging device.

In an embodiment of the invention, the plurality of ultra-wideband transceivers of the source module are arranged spaced apart from each other by a predefined distance, and the plurality of ultra-wideband transceivers of the detector module are arranged spaced apart from each other by a predefined distance.

In an embodiment of the invention, the control unit is configured for determining a current position and/or orientation of the X-ray source of the mobile X-ray imaging device with respect to the X-ray detector of the mobile X-ray imaging device based on the determined mutual distances.

In an embodiment of the invention, the data corresponding to the determined mutual distances comprises the current position and/or orientation.

In an embodiment of the invention, the control unit is configured for receiving a target position and/or orientation of the X-ray source with respect to the X-ray detector of the mobile X-ray imaging device, and calculating a deviation of the current position and/or orientation from the target position and/or orientation, wherein the data corresponding to the determined mutual distances comprises the deviation of the current position and/or orientation from the target position and/or orientation, and/or instructions to a user of the mobile X-ray imaging device how to decrease the deviation.

In an embodiment of the invention, the source module and/or the detector module comprises a built-in energy supply for the ultra-wideband transceivers.

In an embodiment of the invention, the source module is configured to be removably attached to the X-ray source of the mobile X-ray imaging device and/or the detector module is configured to be removably attached to the X-ray detector of the mobile X-ray imaging device.

According to another aspect of the invention, there is provided a mobile X-ray imaging device comprising the system for positioning an X-ray source and an X-ray detector of a mobile X-ray imaging device according to any of the preceding embodiments.

According to another aspect of the invention, there is provided a method for positioning an X-ray source and an X-ray detector of a mobile X-ray imaging device. The method comprises the steps of providing a mobile X-ray imaging device comprising the system for positioning an X-ray source and an X-ray detector of a mobile X-ray imaging device according to any of the preceding embodiments, positioning the X-ray detector of the mobile X-ray imaging device with the detector module being attached to the X-ray detector close to a patient to be imaged, and positioning the X-ray source of the mobile X-ray imaging device with the source module being attached to the X-ray source in front of the patient. The method comprises further the steps of correcting the position and/or orientation of the X-ray source of the mobile X-ray imaging device based on the data corresponding to the determined mutual distances of the plurality of ultra-wideband transceivers of the source module to each of the plurality of ultra-wideband transceivers of the detector module, and triggering the exposure of the X-ray detector with the X-ray source of the mobile X-ray imaging device.

Thus, an operation process of the proposed system for positioning an X-ray source and an X-ray detector may be as follows: A nurse or technician places an X-ray detector plate with attached UWB transceivers behind a patient. The mobile X-ray imaging machine with UWB transceivers placed at the source head is situated in front of the patient for acquiring an X-ray image of the patient. Preferably, a chest X-ray image can be acquired through a window separating the patient from the X-ray source. The technician may select a desirable positioning scheme at a user interface and accurately place the source head of the mobile X-ray imaging device taking into account the determined position and orientation of the X-ray source and detector as well as suggestions and instructions generated by the proposed system based on the measured distances between the ultra-wideband transceivers. After the desired position has been reached, the frames with the ultra-wideband transceivers can optionally be removed from the detector plate and/or the mobile X-ray source head. After the nurse and the technologist retreated to a safe distance, the exposure can be triggered.

In an embodiment of the invention, the method comprises the step of removing the source module from the X-ray source and/or removing the detector module from the X-ray detector before triggering the exposure.
Thus, the benefits provided by any of the above aspects equally apply to all of the other aspects and vice versa.

In summary, the invention relates to a system for positioning an X-ray source and an X-ray detector of a mobile X-ray imaging device. A source module and a detector module are mechanically connected to the X-ray source and the X-ray detector of the mobile X-ray imaging device, respectively. Each of the source module and the detector module comprise a plurality of ultra-wideband transceivers. By determining mutual distances of each of the plurality of ultra-wideband transceivers of the source module to each of the plurality of ultra-wideband transceivers of the detector module, the relative position of the X-ray source and the X-ray detector are determined, and instructions can be provided to a user of the mobile X-ray imaging device how to improve the relative position.

One of the advantages of embodiments of the present invention for positioning an X-ray source and an X-ray detector of a mobile X-ray imaging device is that an operator of the mobile X-ray imaging device is supported in locating the mobile X-ray collimator head relatively to the detector plate to obtain an appropriate diagnostic X-ray image of the patient. Another advantage is that it may eliminate a need for snapshot re-acquiring and thus reduces time delays and increases patient throughput. Another advantage is that it may decrease the effective radiation dose applied to the patients and the hospital personnel.

These advantages are non-limiting and other advantages may be envisioned within the context of the present application.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a schematic setup of a system for positioning an X-ray source and an X-ray detector of a mobile X-ray imaging device according to an embodiment of the invention.
Fig. 2 shows a schematic setup of a mobile X-ray imaging device system comprising the system for positioning an X-ray source and an X-ray detector of a mobile X-ray imaging device according to an embodiment of the invention.
Fig. 3 shows a block diagram of a method for positioning an X-ray source and an X-ray detector of a mobile X-ray imaging device according to an embodiment of the invention.

### DETAILED DESCRIPTION OF EMBODIMENTS

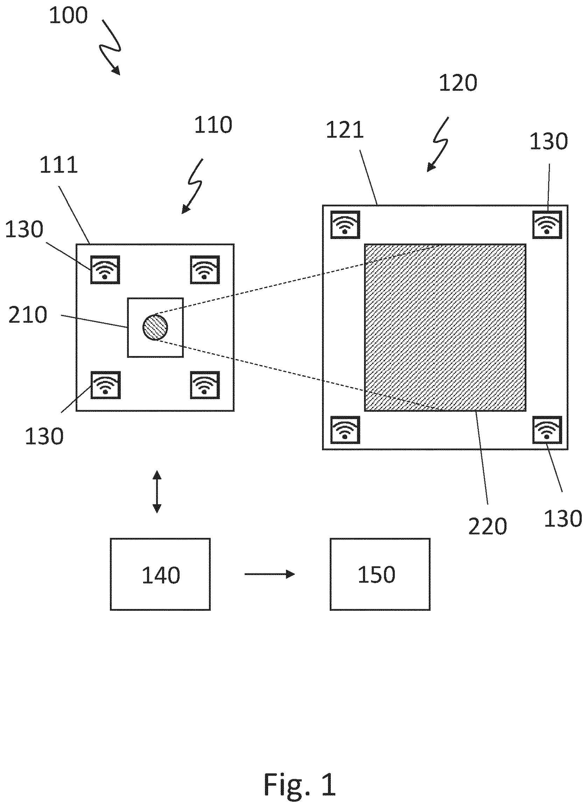

Fig. 1 shows a schematic setup of a system 100 for positioning an X-ray source 210 and an X-ray detector 220 of a mobile X-ray imaging device 200 according to an embodiment of the invention. A mobile X-ray source 210 is provided with a source module 110 connected to the X-ray source 210. The source module 110 comprises a source frame 111 and a plurality of ultra-wideband transceivers 130 connected to the source frame 111. In this embodiment, four ultra-wideband transceivers 130 are comprised in the source module. Similarly, in this embodiment, four ultra-wideband transceivers 130 are connected via a detector frame 121 of a detector module 120 to the X-ray detector 220 of the mobile X-ray imaging device 200. Although at least three ultra-wideband transceivers 130 at each of the X-ray source and the X-ray detector would be sufficient, four transceivers are shown attached to each corner of the source frame 11 and the detector frame 121, respectively. This might increase the precision of the positioning. A control unit 140 communicates with the ultra-wideband transceivers 130, preferably wirelessly, and determines mutual distances of each of the plurality of ultra-wideband transceivers 130 of the source module 110 to each of the plurality of ultra-wideband transceivers 130 of the detector module 120. An interface unit displays data corresponding to the determined mutual distances.

Thus, the proposed invention can implement smart guided positioning for mobile X-ray examinations, and may help an operator to correctly locate the mobile X-ray collimator head of the X-ray source 210 relative to the detector plate of the X-ray detector 220 to obtain high quality X-ray images. Each of the ultra-wideband transceivers 130 may be configured for determining a mutual distance to at least one another ultra-wideband transceiver 130. By taking into account the positions of corners or predetermined points of X-ray source and X-ray detector where the transceivers are located arranged spaced apart from each other by a predefined distance, a relative position and orientation of the X-ray source 210 with respect to a plane position of the X-ray detector 220 can be calculated based on the mutual distances of the ultra-wideband transceivers 130. In an embodiment, a wireless tablet including a data wireless streaming module can instruct an operator how to correction the position and/or orientation of the X-ray source 210 in order to correctly illuminate the X-ray detector 220. This may eliminate the need for snapshot re-acquiring, which saves time, prevents overdose to patients and increases the accuracy of diagnosis.

Advantageously, the determination of the mutual distances of pairs of the ultra-wideband transceivers 130 also in case of through-glass chest radiography, i.e. if the X-ray source 210 and the X-ray detector are separated from each other by a glass window, for example. This might be the case if an infected patient is in an isolated room. Camera-based approaches, including infra-red depth cameras, ultrasonic and laser distance meters, cannot work correctly with obstacles like glass windows. Along with this, a new approach based on ultra-wideband technology is proposed. With this technology, a standard deviation of distance estimation of less than 3 cm at distances of up to 10 meters can be achieved.

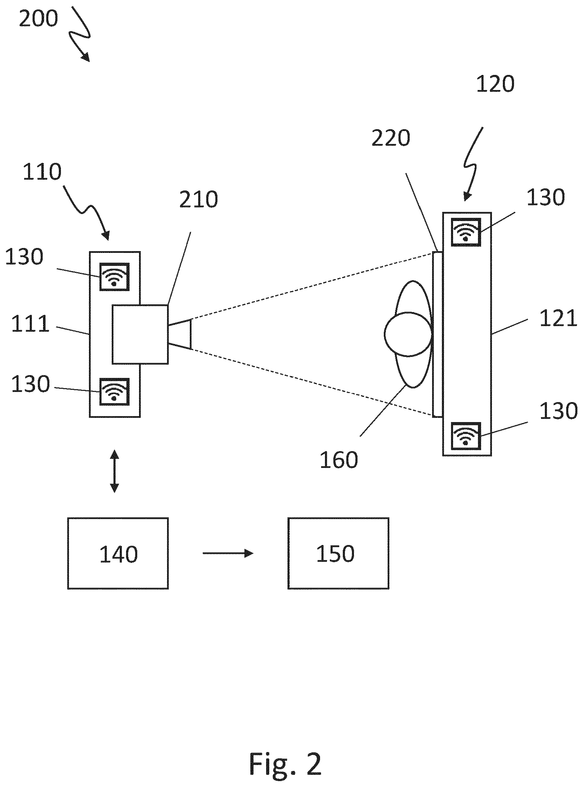

With reference to Fig. 2 showing a schematic setup of a mobile X-ray imaging device 200 comprising the system 100 for positioning an X-ray source 210 and an X-ray detector 220, a patient 160 may be located in an isolated room (not shown). Thus, a through-glass chest X-ray screening pipeline for mobile X-ray machines is shown. The distance from the X-ray source 210 to the X-ray detector 220 producing the image varies for mobile X-ray imaging devices 200 usually from 2 to 4 meters, which is greater than for a conventional X-ray imaging method with about 1.8 meters. This may be due to the patient 160 position like sitting or lying, the length of the stretcher and the depth of the glass wall. So at the distance of 3 meters, a mobile X-ray emitter tilt of only 1 degree will provide a vertical shift of 5.2 cm, which is a significant part of the overall detector height, which leads to producing a mispositioned X-ray image with wrong body areas capture. Device realignment and new image acquisition are required, which is even unfavorable compared to a conventional pipeline, because of a higher power of the X-ray source 210 required for mobile X-ray imaging. For example, with a glass obstacle, the intensity of the X-ray source may need to be increase by a factor of 2 to 6 compared to conventional radiography observations with a stationary X-ray system.

The control unit 140 is in communication with the ultra-wideband transceivers and determines a current position and/or orientation of the X-ray source 210 of the mobile X-ray imaging device 200 with respect to the X-ray detector 220 of the mobile X-ray imaging device 200 based on the determined mutual distances. Thus, the data corresponding to the determined mutual distances that is provided by the interface unit 150 can comprise the current position and/or orientation of the source and the detector, preferably relative to each other.

The control unit may be configured for receiving a target position and/or orientation of the X-ray source 210 with respect to the X-ray detector 220 of the mobile X-ray imaging device. By comparing the target position with the determined current position, a deviation of the current position and/or orientation from the target position and/or orientation can be calculated. The data provided by the interface unit may thus comprise the calculated deviation. In addition or alternatively, instructions to a user of the mobile X-ray imaging device 200 can be provided how to decrease the deviation.

By the direct and precise positioning of the X-ray source and the X-ray detector, it is ensured that unnecessary retakes of the image due to an initial mal-positioning are avoided. Thus, the invention can provide several advantages. From the perspective of the patient, every new image acquisition means additional time delays which have high cost for a possibly sore patient. With this invention, one snapshot is sufficient. Further, the whole procedure of acquiring a good image takes less time, which means that a next patient will get attention earlier. From the clinical personnel perspective, an excessive accumulation of the effective radiation dose can be reduced. During the pandemics with a huge number of patients early interruption of work may occur because of reaching the radiation norms of the clinical personnel. By reducing the number of unnecessary retakes, this can be avoided. From the medical institution perspective, faster care of the patient will save personnel time, which means money savings for the hospital. In addition, reducing the number of image acquisitions for the X-ray machine will increase the lifetime of the X-ray tube in it, which leads to costs savings.

Preferably, the source module 110 and/or the detector module 120 comprise a built-in energy supply for the ultra-wideband transceivers. This may allow upgrading existing X-ray imaging devices. In addition, it may facilitate removing the source module 110 from the X-ray source 210 of the mobile X-ray imaging device and/or the detector module 120 from the X-ray detector 220 of the mobile X-ray imaging device before triggering the exposure. This may ensure patient/equipment safety if the frames with UWB transceivers are removed from the detector and the X-ray head before the exposure procedure.

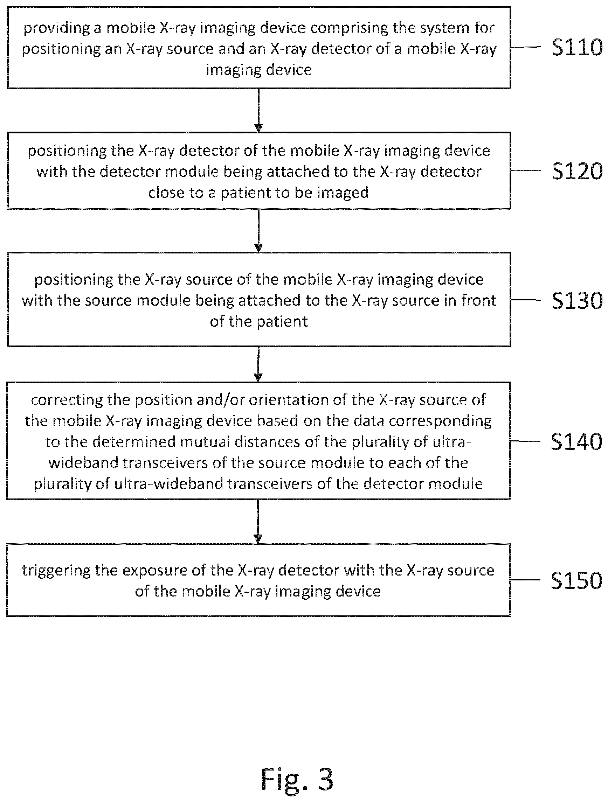

With reference to Fig. 3, a block diagram of a method for positioning an X-ray source 210 and an X-ray detector 220 of a mobile X-ray imaging device 200 according to an embodiment of the invention is shown. The method comprises the step S110 of providing a mobile X-ray imaging device 200 comprising the system 100 for positioning an X-ray source 210 and an X-ray detector 220 of a mobile X-ray imaging device, and the steps S120 and S130 of positioning the X-ray detector of the mobile X-ray imaging device with the detector module 120 being attached to the X-ray detector 220 close to a patient 160 to be imaged, and positioning the X-ray source 210 of the mobile X-ray imaging device with the source module 110 being attached to the X-ray source in front of the patient 160. The method comprises further the step S 140 of correcting the position and/or orientation of the X-ray source 210 of the mobile X-ray imaging device based on the data corresponding to the determined mutual distances of the plurality of ultra-wideband transceivers 130 of the source module 110 to each of the plurality of ultra-wideband transceivers 130 of the detector module 120, and the step S150 of triggering the exposure of the X-ray detector with the X-ray source of the mobile X-ray imaging device.

Thus, an operation process of the proposed system for positioning an X-ray source and an X-ray detector may be as follows: A nurse or technician places an X-ray detector plate with attached UWB transceivers behind a patient. The mobile X-ray imaging machine with UWB transceivers placed at the source head is situated in front of the patient for acquiring an X-ray image of the patient. Preferably, a chest X-ray image can be acquired through a window separating the patient from the X-ray source. The technician may select a desirable positioning scheme at a user interface and accurately place the source head of the mobile X-ray imaging device taking into account the determined position and orientation of the X-ray source and detector as well as suggestions and instructions generated by the proposed system based on the measured distances between the ultra-wideband transceivers. After the desired position has been reached, the frames with the ultra-wideband transceivers can optionally be removed from the detector plate and/or the mobile X-ray source head. After the nurse and the technologist retreated to a safe distance, the exposure can be triggered.

Thus, in a preferred embodiment of the invention, a mobile DXR system comprising a movable X-ray source head including a collimator head and a wireless X-ray detector is equipped with UWB transceivers with included energy sources. Special modules with UWB transceivers, e.g. removable plastic frames with internal low power batteries and at least 3 transceivers in the head of a mobile X-ray device and at least 3 transceivers on the detector are provided and connected to the components of the mobile DXR system. Additionally, the real-time calculation of the mutual distances between detector plane and mobile X-ray head is performed. A user interface of the mobile X-ray machine, an external display as part of the removable frame with UWB transceivers placed on mobile X-ray, or a technologist's tablet can be used to directly stream data corresponding to the calculated mutual distances. The source and detector position can be continuously displayed, and a desirable location scheme can be selected, depending on patient position, exploring body area, obstacle type, etc. The operator or technician can thus be provided with positioning recommendations.

The proposed method is in principle applicable to every hospital with mobile X-ray machines. Low power requirements for the transceivers allow to use autonomous low energy sources like small batteries placed in removable frames with UWB transceivers attached to equipment already existing in hospitals.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: system
- 110: source module
- 111: source frame
- 120: detector module
- 121: detector frame
- 130: ultra-wideband transceiver
- 140: control unit
- 150: interface unit
- 160: patient
- 200: X-ray imaging device
- 210: X-ray source
- 220: X-ray detector

## Claims

1. A system (100) for positioning an X-ray source (210) and an X-ray detector (220) of a mobile X-ray imaging device (200), the system comprising:
a source module (110) configured to be mechanically connected to the X-ray source (210) of the mobile X-ray imaging device (200), the source module (110) comprising a plurality of ultra-wideband transceivers (130);
a detector module (120) configured to be mechanically connected to the X-ray detector (220) of the mobile X-ray imaging device (200), the detector module (120) comprising a plurality of ultra-wideband transceivers (130);
a control unit (140) configured for communicating with the ultra-wideband transceivers (130) and for determining mutual distances of each of the plurality of ultra-wideband transceivers (130) of the source module (110) to each of the plurality of ultra-wideband transceivers (130) of the detector module (120); and
an interface unit (150) configured for displaying data corresponding to the determined mutual distances.

2. The system (100) according to claim 1, wherein each of the ultra-wideband transceivers (130) is configured for determining a mutual distance to at least one another ultra-wideband transceiver (130).

3. The system (100) according to any of claims 1 or 2, wherein the plurality of ultra-wideband transceivers (130) of the source module (110) comprises at least three ultra-wideband transceivers (130), and wherein the plurality of ultra-wideband transceivers (130) of the detector module (120) comprises at least three ultra-wideband transceivers (130).

4. The system (100) according to any of the preceding claims, wherein the source module (110) comprises a source frame (111) configured for receiving the plurality of ultra-wideband transceivers (130), and wherein the source frame (111) is configured to be attached to the X-ray source (210) of the mobile X-ray imaging device (200).

5. The system (100) according to any of the preceding claims, wherein the detector module (120) comprises a detector frame (121) configured for receiving the plurality of ultra-wideband transceivers (130), and wherein the detector frame (121) is configured for receiving the X-ray detector (220) of the mobile X-ray imaging device (200).

6. The system (100) according to any of the preceding claims, wherein the plurality of ultra-wideband transceivers (130) of the source module (110) are arranged spaced apart from each other by a predefined distance, and wherein the plurality of ultra-wideband transceivers (130) of the detector module (120) are arranged spaced apart from each other by a predefined distance.

7. The system (100) according to any of the preceding claims, wherein the control unit (140) is configured for determining a current position and/or orientation of the X-ray source (210) of the mobile X-ray imaging device with respect to the X-ray detector (220) of the mobile X-ray imaging device based on the determined mutual distances.

8. The system (100) according to claim 7, wherein the data corresponding to the determined mutual distances comprises the current position and/or orientation.

9. The system (100) according to any of claims 7 or 8, wherein the control unit (140) is configured for
receiving a target position and/or orientation of the X-ray source (210) with respect to the X-ray detector (220) of the mobile X-ray imaging device (200), and
calculating a deviation of the current position and/or orientation from the target position and/or orientation,
wherein the data corresponding to the determined mutual distances comprises the deviation of the current position and/or orientation from the target position and/or orientation, and/or instructions to a user of the mobile X-ray imaging device (200) how to decrease the deviation.

10. The system (100) according to any of the preceding claims, wherein the source module (110) and/or the detector module (120) comprises a built-in energy supply for the ultra-wideband transceivers (130).

11. The system (100) according to any of the preceding claims, wherein the source module (110) is configured to be removably attached to the X-ray source (210) of the mobile X-ray imaging device and/or wherein the detector module (120) is configured to be removably attached to the X-ray detector (220) of the mobile X-ray imaging device.

12. A mobile X-ray imaging device (200) comprising the system (100) for positioning an X-ray source (210) and an X-ray detector (220) of a mobile X-ray imaging device (200) according to any of claims 1 to 11.

13. A method for positioning an X-ray source (210) and an X-ray detector (220) of a mobile X-ray imaging device (200), the method comprising the steps of:
providing a mobile X-ray imaging device (200) comprising the system (100) for positioning an X-ray source (210) and an X-ray detector (220) of a mobile X-ray imaging device (200) according to any of claims 1 to 11;
positioning the X-ray detector (220) of the mobile X-ray imaging device with the detector module (120) being attached to the X-ray detector close to a patient (160) to be imaged;
positioning the X-ray source (210) of the mobile X-ray imaging device with the source module (110) being attached to the X-ray source in front of the patient (160);
correcting the position and/or orientation of the X-ray source (210) of the mobile X-ray imaging device (200) based on the data corresponding to the determined mutual distances of the plurality of ultra-wideband transceivers (130) of the source module (110) to each of the plurality of ultra-wideband transceivers (130) of the detector module (120); and
triggering the exposure of the X-ray detector (220) with the X-ray source (210) of the mobile X-ray imaging device (200).

14. The method according to claim 13, wherein the method comprises the step of removing the source module (110) from the X-ray source (210) and/or removing the detector module (120) from the X-ray detector (220) before triggering the exposure.

## Patentansprüche

1. System (100) zur Positionieren einer Röntgenquelle (210) und eines Röntgendetektors (220) einer mobilen Röntgenbildgebungsvorrichtung (200), wobei das System Folgendes umfasst:
ein Quellenmodul (110), das so konfiguriert ist, dass es mechanisch mit der Röntgenquelle (210) der mobilen Röntgenbildgebungsvorrichtung (200) verbunden werden kann, wobei das Quellenmodul (110) eine Vielzahl von Ultrabreitband-Transceivern (130) umfasst;
ein Detektormodul (120), das so konfiguriert ist, dass es mechanisch mit dem Röntgendetektor (220) der mobilen Röntgenbildgebungsvorrichtung (200) verbunden werden kann, wobei das Detektormodul (120) eine Vielzahl von Ultrabreitband-Transceivern (130) umfasst;
eine Steuereinheit (140), die zur Kommunikation mit den Ultrabreitband-Transceivern (130) und zur Ermittlung der gegenseitigen Entfernungen jedes der Vielzahl von Ultrabreitband-Transceivern (130) des Quellenmoduls (110) zu jedem der Vielzahl von Ultrabreitband-Transceivern (130) des Detektormoduls (120) konfiguriert ist; und
eine Schnittstelleneinheit (150), die zur Anzeige von Daten entsprechend den ermittelten gegenseitigen Abständen konfiguriert ist.

2. System (100) nach Anspruch 1, wobei jeder der Ultrabreitband-Transceiver (130) zur Ermittlung eines gegenseitigen Abstands zu mindestens einem anderen Ultrabreitband-Transceiver (130) konfiguriert ist.

3. System (100) nach einem der Ansprüche 1 oder 2, wobei die Vielzahl von Ultrabreitband-Transceivern (130) des Quellenmoduls (110) mindestens drei Ultrabreitband-Transceiver (130) umfasst und wobei die Vielzahl von Ultrabreitband-Transceivern (130) des Detektormoduls (120) mindestens drei Ultrabreitband-Transceiver (130) umfasst.

4. System (100) nach einem der vorstehenden Ansprüche, wobei das Quellenmodul (110) einen Quellenrahmen (111) umfasst, der zum Empfang der Vielzahl von Ultrabreitband-Transceivern (130) konfiguriert ist, und wobei der Quellenrahmen (111) so konfiguriert ist, dass er an der Röntgenquelle (210) der mobilen Röntgenbildgebungsvorrichtung (200) angebracht werden kann.

5. System (100) nach einem der vorstehenden Ansprüche, wobei das Detektormodul (120) einen Detektorrahmen (121) umfasst, der zur Aufnahme der Vielzahl von Ultrabreitband-Transceivern (130) konfiguriert ist, und wobei der Detektorrahmen (121) zur Aufnahme des Röntgendetektors (220) der mobilen Röntgenbildgebungsvorrichtung (200) konfiguriert ist.

6. System (100) nach einem der vorstehenden Ansprüche, wobei die Vielzahl von Ultrabreitband-Transceivern (130) des Quellenmoduls (110) in einem vordefinierten Abstand zueinander angeordnet ist und wobei die Vielzahl von Ultrabreitband-Transceivern (130) des Detektormoduls (120) in einem vordefinierten Abstand zueinander angeordnet ist.

7. System (100) nach einem der vorstehenden Ansprüche, wobei die Steuereinheit (140) zur Ermittlung einer aktuellen Position und/oder Ausrichtung der Röntgenquelle (210) der mobilen Röntgenbildgebungsvorrichtung in Bezug auf den Röntgendetektor (220) der mobilen Röntgenbildgebungsvorrichtung basierend auf den ermittelten gegenseitigen Abständen konfiguriert ist.

8. System (100) nach Anspruch 7, wobei die den ermittelten gegenseitigen Abständen entsprechenden Daten die aktuelle Position und/oder Ausrichtung umfassen.

9. System (100) nach einem der Ansprüche 7 oder 8, wobei die Steuereinheit (140) konfiguriert ist zum:
Empfangen einer Zielposition und/oder -ausrichtung der Röntgenquelle (210) in Bezug auf den Röntgendetektor (220) der mobilen Röntgenbildgebungsvorrichtung (200), und
Berechnen einer Abweichung der aktuellen Position und/oder Ausrichtung von der Zielposition und/oder -ausrichtung,
wobei die den ermittelten gegenseitigen Abständen entsprechenden Daten die Abweichung der aktuellen Position und/oder Ausrichtung von der Zielposition und/oder -ausrichtung, und/oder Anweisungen an einen Benutzer der mobilen Röntgenbildgebungsvorrichtung (200), wie die Abweichung verringert werden kann, umfassen.

10. System (100) nach einem der vorstehenden Ansprüche, wobei das Quellenmodul (110) und/oder das Detektormodul (120) eine eingebaute Energieversorgung für die Ultrabreitband-Transceiver (130) umfasst.

11. System (100) nach einem der vorstehenden Ansprüche, wobei das Quellenmodul (110) so konfiguriert ist, dass es abnehmbar an der Röntgenquelle (210) der mobilen Röntgenbildgebungsvorrichtung befestigt werden kann, und/oder wobei das Detektormodul (120) so konfiguriert ist, dass es abnehmbar an den Röntgendetektor (220) der mobilen Röntgenbildgebungsvorrichtung angebracht werden kann.

12. Mobile Röntgenbildgebungsvorrichtung (200), umfassend das System (100) zum Positionieren einer Röntgenquelle (210) und eines Röntgendetektors (220) einer mobilen Röntgenbildgebungsvorrichtung (200) nach einem der Ansprüche 1 bis 11.

13. Verfahren zum Positionieren einer Röntgenquelle (210) und eines Röntgendetektors (220) einer mobilen Röntgenbildgebungsvorrichtung (200), wobei das Verfahren die folgenden Schritte umfasst:
Bereitstellen einer mobilen Röntgenbildgebungsvorrichtung (200), umfassend das System (100) zum Positionieren einer Röntgenquelle (210) und eines Röntgendetektors (220) einer mobilen Röntgenbildgebungsvorrichtung (200) nach einem der Ansprüche 1 bis 11;
Positionieren des Röntgendetektors (220) der mobilen Röntgenbildgebungsvorrichtung, wobei das Detektormodul (120) am Röntgendetektor in der Nähe des zu untersuchenden Patienten (160) angebracht wird;
Positionieren der Röntgenquelle (210) der mobilen Röntgenbildgebungsvorrichtung, wobei das Quellenmodul (110) an der Röntgenquelle vor dem Patienten (160) angebracht wird;
Korrigieren der Position und/oder Ausrichtung der Röntgenquelle (210) der mobilen Röntgenbildgebungsvorrichtung (200) basierend auf den Daten, die den ermittelten gegenseitigen Abständen der Vielzahl von Ultrabreitband-Transceivern (130) des Quellenmoduls (110) zu jedem der Vielzahl von Ultrabreitband-Transceivern (130) des Detektormoduls (120) entsprechen; und
Auslösen der Belichtung des Röntgendetektors (220) mit der Röntgenquelle (210) der mobilen Röntgenbildgebungsvorrichtung (200).

14. Verfahren nach Anspruch 13, wobei das Verfahren den Schritt des Entfernens des Quellenmoduls (110) von der Röntgenquelle (210) und/oder des Entfernens des Detektormoduls (120) vor Auslösung der Belichtung vom Röntgendetektor (220) umfasst.

## Revendications

1. Système (100) pour positionner une source de rayons X (210) et un détecteur de rayons X (220) d'un dispositif d'imagerie radiologique mobile (200), le système comprenant :
un module source (110) configuré pour être connecté mécaniquement à la source de rayons X (210) du dispositif d'imagerie radiologique mobile (200), le module source (110) comprenant une pluralité d'émetteurs-récepteurs à ultra-large bande (130) ;
un module détecteur (120) configuré pour être connecté mécaniquement au détecteur de rayons X (220) du dispositif d'imagerie radiologique mobile (200), le module détecteur (120) comprenant une pluralité d'émetteurs-récepteurs à ultra-large bande (130) ;
une unité de commande (140) configurée pour communiquer avec les émetteurs-récepteurs à ultra-large bande (130) et pour déterminer des distances mutuelles de chacun de la pluralité d'émetteurs-récepteurs à ultra-large bande (130) du module source (110) à chacun des émetteurs-récepteurs à ultra-large bande (130) du module détecteur (120) ; et
une unité d'interface (150) configurée pour afficher des données correspondant aux distances mutuelles déterminées.

2. Système (100) selon la revendication 1, dans lequel chacun des émetteurs-récepteurs à ultra-large bande (130) est configuré pour déterminer une distance mutuelle par rapport à au moins un autre émetteur-récepteur à ultra-large bande (130).

3. Système (100) selon l'une quelconque des revendications 1 ou 2, dans lequel la pluralité d'émetteurs-récepteurs à ultra-large bande (130) du module source (110) comprennent au moins trois émetteurs-récepteurs à ultra-large bande (130), et dans lequel la pluralité d'émetteurs-récepteurs à ultra-large bande (130) du module détecteur (120) comprennent au moins trois émetteurs-récepteurs à ultra-large bande (130).

4. Système (100) selon l'une quelconque des revendications précédentes, dans lequel le module source (110) comprend un cadre source (111) configuré pour recevoir la pluralité d'émetteurs-récepteurs à ultra-large bande (130), et dans lequel le cadre source (111) est configuré pour être fixé à la source de rayons X (210) du dispositif d'imagerie radiologique mobile (200).

5. Système (100) selon l'une quelconque des revendications précédentes, dans lequel le module détecteur (120) comprend un cadre détecteur (121) configuré pour recevoir la pluralité d'émetteurs-récepteurs à ultra-large bande (130), et dans lequel le cadre détecteur (121) est configuré pour recevoir le détecteur de rayons X (220) du dispositif d'imagerie radiologique mobile (200).

6. Système (100) selon l'une quelconque des revendications précédentes, dans lequel la pluralité d'émetteurs-récepteurs à ultra-large bande (130) du module source (110) sont agencés à distance les uns des autres d'une distance prédéfinie, et dans lequel la pluralité d'émetteurs-récepteurs à ultra-large bande (130) du module détecteur (120) sont disposés à distance les uns des autres d'une distance prédéfinie.

7. Système (100) selon l'une quelconque des revendications précédentes, dans lequel l'unité de commande (140) est configurée pour déterminer une position et/ou une orientation actuelles de la source de rayons X (210) du dispositif d'imagerie radiographique mobile par rapport au détecteur de rayons X (220) du dispositif d'imagerie radiographique mobile en fonction des distances mutuelles déterminées.

8. Système (100) selon la revendication 7, dans lequel les données correspondant aux distances mutuelles déterminées comprennent la position et/ou l'orientation actuelles.

9. Système (100) selon l'une quelconque des revendications 7 ou 8, dans lequel l'unité de commande (140) est configurée pour
recevoir une position et/ou une orientation cibles de la source de rayons X (210) par rapport au détecteur de rayons X (220) du dispositif d'imagerie radiographique mobile (200), et
calculer un écart entre la position et/ou l'orientation actuelles par rapport à la position et/ou à l'orientation cibles,
dans lequel les données correspondant aux distances mutuelles déterminées comprennent l'écart de la position et/ou de l'orientation actuelles par rapport à la position et/ou à l'orientation cibles, et/ou aux instructions à un utilisateur du dispositif d'imagerie radiographique mobile (200) sur la manière de réduire l'écart.

10. Système (100) selon l'une quelconque des revendications précédentes, dans lequel le module source (110) et/ou le module détecteur (120) comprennent une alimentation en énergie intégrée pour les émetteurs-récepteurs à ultra-large bande (130).

11. Système (100) selon l'une quelconque des revendications précédentes, dans lequel le module source (110) est configuré pour être fixé de manière amovible à la source de rayons X (210) du dispositif d'imagerie radiologique mobile et/ou dans lequel le module détecteur (120) est configuré pour être fixé de manière amovible au détecteur de rayons X (220) du dispositif d'imagerie radiographique mobile.

12. Dispositif d'imagerie radiographique mobile (200) comprenant le système (100) pour positionner une source de rayons X (210) et un détecteur de rayons X (220) d'un dispositif d'imagerie radiographique mobile (200) selon l'une quelconque des revendications 1 à 11.

13. Procédé de positionnement d'une source de rayons X (210) et d'un détecteur de rayons X (220) d'un dispositif d'imagerie radiologique mobile (200), le procédé comprenant les étapes de :
fourniture d'un dispositif d'imagerie radiographique mobile (200) comprenant le système (100) pour positionner une source de rayons X (210) et un détecteur de rayons X (220) d'un dispositif d'imagerie radiographique mobile (200) selon l'une quelconque des revendications 1 à 11 ;
positionnement du détecteur de rayons X (220) du dispositif d'imagerie radiographique mobile avec le module détecteur (120) fixé au détecteur de rayons X près d'un patient (160) à imager ;
positionnement de la source de rayons X (210) du dispositif d'imagerie radiographique mobile avec le module source (110) fixé à la source de rayons X devant le patient (160) ;
correction de la position et/ou de l'orientation de la source de rayons X (210) du dispositif d'imagerie radiographique mobile (200) basée sur les données correspondant aux distances mutuelles déterminées de la pluralité d'émetteurs-récepteurs à ultra-large bande (130) du module source (110) à chacune des pluralités d'émetteurs-récepteurs à ultra-large bande (130) du module détecteur (120) ; et
déclenchement de l'exposition du détecteur de rayons X (220) avec la source de rayons X (210) du dispositif d'imagerie radiographique mobile (200).

14. Procédé selon la revendication 13, dans lequel le procédé comprend l'étape de retrait du module source (110) de la source de rayons X (210) et/ou de retrait du module détecteur (120) du détecteur de rayons X (220) avant le déclenchement de l'exposition.
